# FASCICULE DE BREVET EUROPEEN

(11) **EP 3 166 521 B1**
(45) Date de publication et mention de la délivrance du brevet: **24.01.2024**
(21) Numéro de dépôt: 15753100.5
(22) Date de dépôt: 09.07.2015
(51) Int. Cl.: A61B 17/80

(54) **IMPLANT ET KIT CHIRURGICAL POUR MAINTENIR EN POSITION DES CORPS OSSEUX D'UN PATIENT LES UNS PAR RAPPORT AUX AUTRES**
IMPLANTAT UND CHIRURGISCHES KIT ZUM HALTEN VON KNOCHENKÖRPERN EINES PATIENTEN IN POSITION ZUEINANDER
IMPLANT AND SURGICAL KIT FOR HOLDING BONE BODIES OF A PATIENT IN POSITION WITH RESPECT TO ONE ANOTHER

(30) Priorité: 10.07.2014 FR 1456664
(43) Date de publication de la demande: 17.05.2017
(73) Titulaire: In2Bones, 69130 Ecully (FR)
(72) Inventeur: CHICK, Grégoire, 1201 Geneve (CH); PAPALOÏZOS, Michaël, 1204 Genève (CH)
(74) Mandataire: Cabinet Didier Martin
(86) Numéro de dépôt international: PCT/FR2015/051904
(87) Numéro de publication internationale: WO 2016/005705

(56) Documents cités:
- EP-A2- 2 099 373
- FR-A1- 2 920 960
- US-A1- 2005 234 458
- US-A1- 2007 270 853

## Description

### DOMAINE TECHNIQUE

L'invention se rapporte au domaine général des implants chirurgicaux d'ostéosynthèse, en particulier conçus pour le traitement de fractures ou de fêlures de corps osseux situés notamment dans l'avant-bras.

L'invention concerne plus particulièrement un implant chirurgical destiné à être rapporté sur des corps osseux d'un patient, par exemple de son avant-bras, par l'intermédiaire de moyens de fixation afin de maintenir en position lesdits corps osseux les uns par rapport aux autres, ledit implant chirurgical comportant un corps principal formé par une plaque distale qui se prolonge par une patte proximale, la plaque distale s'étendant sensiblement selon un plan d'extension distal, la patte proximale s'étendant sensiblement le long d'un plan d'extension proximal sécant au plan d'extension distal, la patte proximale et la plaque distale étant reliées par une zone de courbure.

L'invention concerne également un kit chirurgical incluant un implant chirurgical.

### TECHNIQUE ANTERIEURE

Pour améliorer la reconstruction des os de l'avant-bras d'un patient à la suite de certains types de fractures ou de fêlures de l'extrémité distale du radius, il est possible d'avoir recours à un implant chirurgical, généralement désigné par le terme de « *plaque radiale* ». Ce type d'implant chirurgical est le plus souvent formé par une plaque courbée métallique afin que la surface antérieure de la plaque soit de forme convexe pour épouser la forme de la portion extrémale du radius à réparer. La plaque radiale est destinée à être fixée par vissage sur deux parties du radius séparées par fracture de manière à ce que ladite plaque enjambe le trait de fracture et immobilise les deux parties du radius l'une par rapport à l'autre pour favoriser leur fusion.

Si cette solution donne généralement satisfaction et permet l'ostéosynthèse du radius fêlé ou fracturé du patient, il a cependant semblé qu'elle pourrait être améliorée.

En effet, du fait de la très grande variété des morphologies du radius des patients, ces derniers sont susceptibles, dans certains cas, de subir des douleurs au cours du processus d'ostéosynthèse. Quelque fois, le frottement des tissus sur l'implant et notamment sur les arêtes de ce dernier, peut également être source de douleur, en dépit de la faible épaisseur de ce dernier.

Il semble également que la vitesse du processus de reconstruction osseuse, relativement longue, pourrait être améliorée, dans la mesure ou il implique généralement une immobilisation et/ou une protection du poignet, ce qui est généralement inconfortable et gênant pour le patient.

Le document FR 2 920 960 A1 décrit une plaque d'ostéosynthèse qui inclut une partie diaphysaire et une partie métaphysaire élargie, en forme de palette. La partie métaphysaire présente une forme courbe dans un plan perpendiculaire à l'axe longitudinal de la partie diaphysaire, avec une face convexe sur le côté de la plaque destiné à venir au contact de l'os, et la partie métaphysaire comprend deux trous sur son côté destiné à venir en regard de la styloïde, dont un distal et l'autre proximal, ces trous étant aménagés selon des axes parallèles l'un à l'autre, et étant orientés selon un angle d'environ 30 à 35 degrés par rapport au plan dans lequel s'étend la partie diaphysaire. Les deux trous précités comprennent des portions taraudées et les vis destinées à être mises en place dans ces trous comprennent des portions filetées aptes à venir en prise avec ces portions taraudées, de telle sorte que chaque vis puisse être engagée dans l'un de ces trous selon une direction unique, coaxiale au trou.

Le document US 2007/270853 A1 décrit une plaque osseuse pouvant être fixée à un os pour une fixation interne. La plaque osseuse a une surface supérieure et une surface opposée en contact avec l'os et comprend une pluralité de trous traversants filetés pour des attaches osseuses. La plaque osseuse peut comprendre des trous traversants pour le passage de sutures, et des évidements de dégagement des sutures formés sur la surface en contact avec l'os. Chacun des évidements est défini pour se rapprocher d'au moins un des trous de suture pour fournir un dégagement de suture pour un instrument de suture, tel qu'une aiguille de suture incurvée.

### EXPOSE DE L'INVENTION

Les objets assignés à l'invention visent par conséquent à remédier aux inconvénients susmentionnés, en proposant un nouvel implant chirurgical et un nouveau kit chirurgical associé permettant d'accélérer le processus d'ostéosynthèse et la guérison du patient, tout en étant parfaitement toléré.

Un autre objet de l'invention vise à proposer un nouvel implant chirurgical et un nouveau kit chirurgical associé permettant d'obtenir une ostéosynthèse de bonne qualité.

Un autre objet de l'invention vise à proposer un nouvel implant chirurgical et un nouveau kit chirurgical associé permettant de limiter la douleur subie par le patient.

Un autre objet de l'invention vise à proposer un nouvel implant chirurgical et un nouveau kit chirurgical associé permettant de faciliter l'opération d'insertion de l'implant dans le corps du patient.

Un autre objet de l'invention vise à proposer un nouvel implant chirurgical et un nouveau kit chirurgical associé particulièrement polyvalents et s'adaptant à toute morphologie.

Un autre objet de l'invention vise à proposer un nouvel implant chirurgical et un nouveau kit chirurgical associé dont la conception et la fabrication relativement aisée.

Un autre objet de l'invention vise à proposer un nouvel implant chirurgical et un nouveau kit chirurgical associé dont la mise en place dans le corps du patient est facilitée et précise.

Les objets assignés à l'invention sont atteints à l'aide d'un implant chirurgical selon la revendication 1.

Les objets assignés à l'invention sont également atteints à l'aide d'un kit chirurgical selon la revendication 14.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- La figure 1 illustre, selon une vue générale en perspective, un implant chirurgical selon l'invention.
- La figure 2 illustre, selon une vue en perspective, l'implant chirurgical de la figure 1, la vue étant orientée de manière à ce qu'une surface postérieure de ce dernier soit visible au premier plan.
- La figure 3 représente, selon une vue en perspective, l'implant chirurgical des figures 1 et 2, la vue étant orientée de manière à ce qu'un bord distal de ce dernier soit visible au premier plan.
- Les figures 4 et 5 illustrent, selon des vues partielles en perspective, un détail de réalisation de l'implant chirurgical des figures 1 à 3, les vues étant orientées de manière à ce qu'une portion de la surface postérieure et le bord distal soient particulièrement visibles.
- Les figures 6 et 7 représentent, selon des vues partielles en perspective, un détail de réalisation de l'implant chirurgical des figures 1 à 5, les vues étant orientées de manière à ce qu'une portion de la surface antérieure et le bord distal soient particulièrement visibles.
- Les figures 8 et 9 illustrent respectivement, selon des vues en perspective, une vis de fixation de l'implant chirurgical des figures 1 à 7 dont la tête comporte des moyens de rétention, et une vis de fixation dudit implant chirurgical dont la tête est dépourvue de moyens de rétention.
- Les figures 10 et 11 représentent respectivement selon une vue latérale et une vue de dessus, l'implant chirurgical des figures 1 à 7 fixé à des corps osseux d'un patient à l'aide des vis de fixations des figures 8 et 9.
- La figure 12 représente selon une vue en perspective éclatée, un kit chirurgical selon l'invention incluant l'implant chirurgical des figures 1 à 7 et 10 et 11, ainsi qu'un sabot.
- La figure 13 illustre, selon une vue en coupe transversale de l'implant chirurgical des figures précédentes, un exemple de réalisation d'une ouverture de vissage formant un moyen de fixation dudit implant chirurgical.

### MEILLEURE MANIERE DE REALISER L'INVENTION

Les figures mentionnées précédemment sont avantageusement représentées à l'échelle, dans un but illustratif et non limitatif d'une variante préférentielle de l'invention.

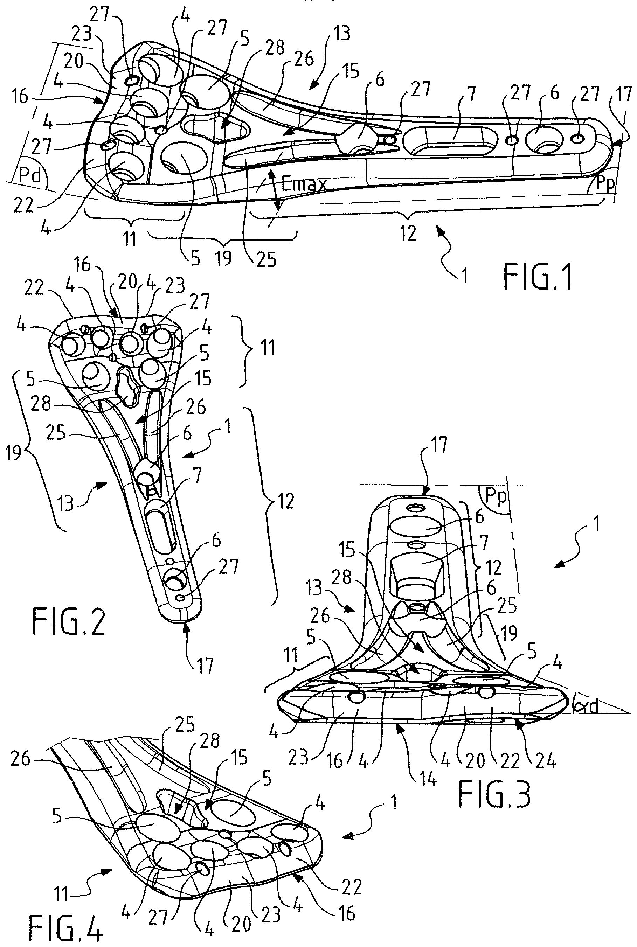

Les figures 1 à 8 illustrent une variante préférentielle d'un implant chirurgical 1 conforme à l'invention. L'implant chirurgical 1, dans sa variante préférée ainsi représentée aux figures, forme un implant d'avant-bras, en l'espèce une plaque radiale pour fracture de l'extrémité distale du radius de l'avant-bras d'un patient. L'invention ne se limite toutefois pas à un implant d'avant bras, et pourra concerner par exemple un implant tibial ou un implant palmaire. En tout état de cause, l'implant chirurgical 1 est prévu pour être implanté dans le corps du patient au cours d'une opération chirurgicale effectuée par exemple sous anesthésie, en particulier à la suite d'une lésion d'un os du patient du type fracture ou fêlure osseuse, pour soigner ou contribuer à soigner ladite lésion au cours d'un processus d'ostéosynthèse. Le patient désigné par l'invention est de préférence un être humain, étant entendu que rien ne s'oppose à l'introduction de l'implant 1 de l'invention dans le corps d'un animal pour soigner des pathologies vétérinaires similaires aux pathologies humaines présentement décrites.

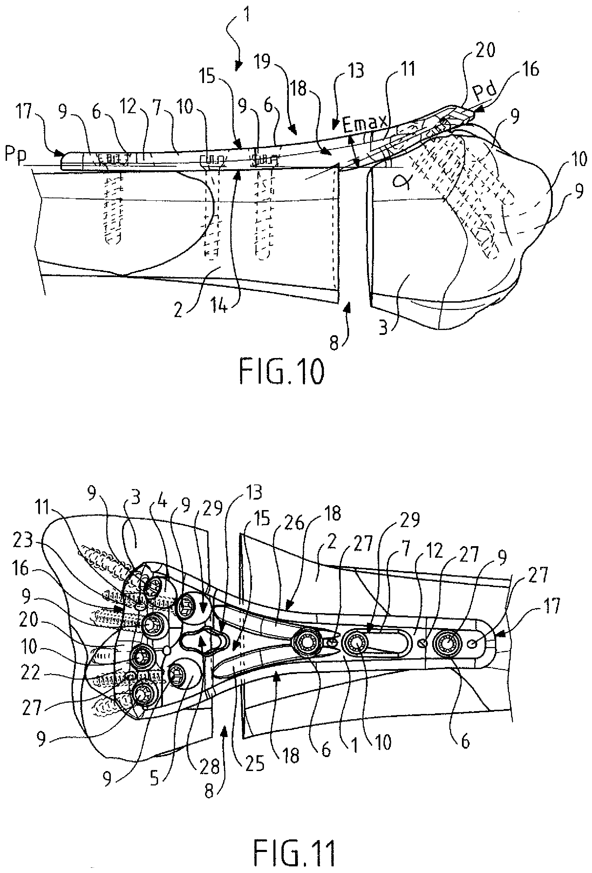

L'implant chirurgical 1 de l'invention est destiné à être rapporté sur des corps osseux 2, 3 d'un patient, par exemple de son avant-bras et/ou de son poignet, par l'intermédiaire de moyens de fixation 4, 5, 6, 7 afin de maintenir en position lesdits corps osseux 2, 3 les uns par rapport aux autres, tel qu'illustré par exemple aux figures 10 et 11, afin en particulier de permettre, ou pour le moins de favoriser, l'ostéosynthèse de ces derniers.

L'implant chirurgical 1 est ainsi conçu pour être introduit dans le corps d'un patient, et pour être fixé temporairement ou définitivement aux corps osseux 2, 3 lésés. On entend par « *moyens de fixation* », des éléments appartenant ou non à l'implant chirurgical 1, et qui permettent de solidariser ce dernier aux corps osseux 2, 3. En l'espèce, l'implant chirurgical 1 comporte un corps principal 13, lequel est destiné à être rapporté sur les corps osseux 2, 3. Les moyens de fixation 4, 5, 6, 7 sont préférentiellement formés par des ouvertures de vissage 4, 5, 6, 7, dans lesquelles sont destinées à être insérées des vis de fixation 9, 10, pour solidariser ledit implant chirurgical 1, en particulier son corps principal 13, avec les corps osseux 2, 3 par vissage desdits vis de fixation 9, 10 dans ces derniers, tel que décrit ci-après. D'autres moyens de fixation pourront être envisagés sans sortir du cadre de l'invention, tels que par exemple des moyens de clipsage, d'agrafage, de clouage ou similaire.

On entend par corps osseux 2,3 des os, des cartilages, des fragments d'os ou de cartilage, voire des tendons, ou une combinaison de ces derniers, de préférence destinés à être fusionnés par ostéosynthèse avec l'aide de l'implant chirurgical 1. L'implant chirurgical 1 étant préférentiellement une plaque radiale, les corps osseux 2, 3 sont préférentiellement formés par un os du radius fêlé ou fracturé de l'avant-bras du patient. En particulier, l'implant chirurgical 1 est destiné à être mis en place sur la partie ventrale de l'avant bras, c'est-à-dire la partie de l'avant bras dans le prolongement de la paume de la main. Bien entendu, l'implant chirurgical 1 de l'invention pourra être mis en place sur d'autres corps osseux du patient, et pour soigner d'autres types de lésions sans sortir du cadre de l'invention.

La mise en place de l'implant chirurgical 1 sur les corps osseux 2, 3 a vocation à sensiblement immobiliser ces derniers les uns par rapport aux autres. En effet, l'implant chirurgical 1 est destiné à être fixé au moins à un premier et un deuxième corps osseux 2, 3, pour relier lesdits corps osseux 2,3 l'un à l'autre en formant un pont apte à maintenir en position relative lesdits corps osseux 2, 3 en enjambant par exemple un trait de fracture 8, ou plus généralement une ligne de séparation des corps osseux 2, 3. L'os fragilisé ou fêlé peut ainsi avantageusement se reconstruire, ses fragments osseux formés par les corps osseux 2,3 étant maintenus en position par l'implant chirurgical 1 dans une position adéquate d'ostéosynthèse.

Il est envisageable bien entendu d'utiliser un tel implant chirurgical 1 pour maintenir, ou contribuer à maintenir, un nombre plus élevé de corps osseux en position les uns par rapport aux autres, par exemple trois, quatre, ou plus.

Selon l'invention, ledit implant chirurgical 1 comporte un corps principal 13. Ce dernier, et plus généralement l'implant chirurgical 1, forme préférentiellement une seule pièce monobloc et unitaire. Bien entendu, l'implant chirurgical 1, et en particulier son corps principal 13, pourra au contraire être le résultat de l'assemblage de plusieurs pièces.

Cette pièce monobloc est préférentiellement réalisée en un matériau radio-transparent, de préférence du polyéther-éther-cétone (PEEK), ce dernier étant optionnellement chargé en carbone, ce qui conduit à un bon compromis entre souplesse et rigidité du corps principal apte à favoriser l'ostéosynthèse et un guérison rapide des lésions osseuses. Alternativement, l'implant chirurgical 1 pourra être réalisé dans un matériau métallique tel que le titane. L'implant chirurgical 1 pourra être réalisé d'un seul tenant par une opération de moulage, suivie éventuellement par une opération d'usinage de la pièce brute moulée, de sorte qu'il est facile à fabriquer

Le corps principal 13 présente avantageusement une surface antérieure 14 destinée à reposer au moins partiellement sur lesdits corps osseux 2, 3, et une surface postérieure 15 opposée à la surface antérieure 14 formant une surface dorsale préférentiellement libre lorsque l'implant chirurgical 1 est en place sur lesdits corps osseux 2, 3.

Selon l'invention, le corps principal 13 est formé par une plaque distale 11 qui se prolonge par une patte proximale 12, tel qu'illustré aux figures 1 à 8. La plaque distale 11 forme une portion du corps principal 13 avantageusement située à une première extrémité de ce dernier, la patte proximale 12 formant l'autre extrémité dans la continuité de ladite plaque distale 11. Le corps principal 13 s'étend préférentiellement longitudinalement entre :
- un bord distal 16, à partir duquel s'étend la plaque distale 11, et
- un bord proximal 17, à partir duquel s'étend la patte proximale 12, et transversalement entre deux bords latéraux 18.

Au sens de l'invention la direction longitudinale de l'implant chirurgical 1 s'étend depuis le bord distal 16 jusqu'au bord proximal 17, alors que la direction transversale s'étend d'un bord latéral 18 à l'autre. Enfin, la direction sagittale représente la troisième direction de l'espace, orthogonale à la fois à la direction longitudinale et transversale.

La surface antérieure 14 s'étend avantageusement depuis la plaque distale 11 jusqu'à la patte proximale 12, de façon continue, douce et longitudinale, sur un côté antérieur de ces dernières. De même, la surface postérieure 15 s'étend préférentiellement depuis la plaque distale 11 jusqu'à la patte proximale 12 de façon continue, douce et longitudinale, sur un côté antérieur de ces dernières.

La plaque distale 11 est avantageusement conçue et conformée pour être rapportée à un corps osseux extrémal, préférentiellement distal, c'est-à-dire situé à l'avant ou à l'antérieur des corps osseux 2, 3, de préférence à une partie distale de l'os du radius, c'est-à-dire au plus près du poignet du patient. La plaque distale 11 de l'invention s'étend sensiblement selon un plan d'extension distal Pd, forme une portion aplatie du corps principal 13, et présente préférentiellement la forme d'une spatule. La plaque distale 11 est prévue pour être fixée à plat sur le corps osseux 3 en reposant sur la surface antérieure 14 (tel qu'illustré aux figures 10 et 11). Le plan d'extension distal Pd s'étend dans la direction transversale, et dans une direction proche de la direction longitudinale.

La patte proximale 12, au contraire de la plaque distale 11, est avantageusement fuselée, en forme de tige ou de manche de spatule, et est plus fine transversalement que cette dernière. La patte proximale 12 est avantageusement rectiligne, mais peut être transversalement courbée. En effet, la patte proximale 12 est préférentiellement destinée à être rapportée sur :
- un corps osseux médian, éloigné des articulations ou des extrémités de l'os considéré, et/ou
- un corps osseux proximal, proche d'une extrémité osseuse opposée au corps osseux extrémal distal.

En particulier, la patte proximale 12 pourra être conçue pour être rapportée sur une partie médiane de l'os du radius du patient. La patte proximale 12, si elle est plus fine que la plaque distale 11, est néanmoins avantageusement légèrement aplatie pour former la surface antérieure 14 par l'intermédiaire elle est destinée à être rapportée sur le corps osseux 2. Selon l'invention, la patte proximale 12 s'étend sensiblement le long d'un plan d'extension proximal Pp, selon l'invention. Le plan d'extension proximal Pp s'étend dans la direction transversale, et dans une direction proche de la direction longitudinale.

Selon l'invention, le plan d'extension proximal Pp sécant au plan d'extension distal Pd, la patte proximale 12 et la plaque distale 11 étant reliées par une zone de courbure 19. Le corps principal 13, selon l'invention, est ainsi légèrement fléchi dans le sens longitudinal, de préférence autour d'un axe transversal, afin de s'adapter à la morphologie osseuse des corps osseux 2, 3, en particulier un os du radius. Le plan d'extension proximal Pp et le plan d'extension distal Pd sont distincts et forment ainsi un léger angle autour de la direction transversale, ce qui est particulièrement visible à la figure 10. La plaque distale 11 est ainsi légèrement relevée par rapport à la patte proximale 12. Le corps principal 13, d'une extrémité à l'autre, comporte ainsi trois sections, savoir la patte proximale 12 suivie par la zone de courbure 19, elle-même suivie par la plaque distale 11. De préférence la plaque distale 11 est inclinée par rapport à la patte proximale 12, de sorte que le plan d'extension distal Pd et le plan d'extension proximal Pp sont sécants l'un par rapport à l'autre de manière à former un angle d'élévation α étant compris entre 10° et 80°, de préférence entre 20° et 30°, et de façon encore plus préférentielle d'environ 25°, afin de s'adapter à la morphologie des corps osseux 2, 3, et notamment à l'inclinaison de la surface de l'os au niveau de l'extrémité de ce dernier par rapport à la surface de l'os en partie médiane de ce dernier.

En d'autres termes, l'angle d'élévation α est assimilable à un angle d'incidence de la plaque distale 11 par rapport à la patte proximale 12. En l'espèce l'angle d'élévation α représente l'écart angulaire entre un prolongement virtuel distal du plan d'extension proximal Pp, et le plan d'extension distal Pd. L'angle d'élévation α témoigne de la légère cambrure terminale de l'implant chirurgical 1 au niveau de sa zone de courbure 19. L'angle d'élévation α est défini tel que représenté par exemple à la figure 10, sous la plaque distale 11.

Dans l'exemple préférentiel illustré aux figures, la zone de courbure 19 est formée par la combinaison de l'extrémité distale de la patte proximale 12 et l'extrémité proximale de la plaque distale 11, et forme une jonction courbe entre les deux. Le corps principal 13 est avantageusement courbé dans une seule direction, en l'espèce la direction longitudinale. Sans sortie du cadre de l'invention, le corps principal 13 pourra présenter d'autres courbures pour s'adapter à la morphologie des corps osseux 2, 3, comme par exemple une torsion longitudinale, ou une flexion autour de l'axe longitudinal.

En tout état de cause, l'implant chirurgical 1, et en particulier le corps principal 13 présente une forme particulièrement adaptée à la morphologie des corps osseux 2, 3 afin de limiter la douleur subie par le patient lorsque l'implant est en place.

Selon une telle conception, la surface antérieure 14 présente de préférence une forme générale convexe particulièrement adaptée à la courbure et la forme de la surface des corps osseux 2, 3, et la surface postérieure 15 une forme générale concave.

Selon une caractéristique importante de l'invention, la zone de courbure 19 présente une épaisseur la plus élevée du corps principal 13. Au sens de l'invention, on entend par « *épaisseur* » la dimension du corps principal 13 dans la direction sagittale, c'est-à-dire au voisinage de l'orthogonale au plan d'extension distal Pd ou au plan d'extension proximal Pp. Au sens de l'invention, le terme « *épaisseur* », lorsqu'il s'applique au corps principal 13 en tant que tel, ne tient pas compte des orifices, ouvertures, fenêtres, concavités, sillons et similaires, ménagés dans ledit corps principal 13, sauf mention du contraire.

Selon une telle conception, l'implant chirurgical 1 de l'invention est d'une rigidité supérieure au niveau de la zone de courbure 19 qu'au niveau du bord distal 16 et/ou du bord proximal 17.

Ainsi, l'implant chirurgical 1 présente une souplesse suffisante pour limiter la douleur subie par le patient au niveau des moyens de fixation 4, 5, 6, 7, et pour stimuler l'ostéosynthèse en autorisant un léger déplacement des corps osseux 2, 3 les uns par rapport aux autres. La souplesse de l'implant chirurgical 1 lui permet également de s'adapter à des morphologies variées de corps osseux.

L'implant chirurgical 1 de l'invention présente également une rigidité suffisante pour maintenir et immobiliser efficacement en position les corps osseux 2, 3 les uns par rapport aux autres afin de favoriser la reconstruction selon un positionnement défini, correspondant avantageusement à la position des corps osseux avant fracture ou fêlure. De manière générale, l'implant chirurgical 1 de l'invention permet d'améliorer la vitesse de guérison et de reconstruction de l'os du patient.

La zone de courbure 19 présente avantageusement une épaisseur supérieure à l'épaisseur d'au moins l'une de la plaque distale 11 et de la patte proximale 12. De façon encore plus préférentielle, la zone de courbure 19 présente une épaisseur supérieure aux épaisseurs respectives de la plaque distale 11 et de la patte proximale 12.

Dans la variante préférée représentée aux figures, la zone de courbure 19 comporte un maximum d'épaisseur Emax, l'épaisseur de la zone de courbure 19 étant décroissante à partir du maximum d'épaisseur Emax, d'une part en direction de la plaque distale 11, d'autre part en direction de la patte proximale 12.

De façon préférentielle, la surface antérieure 14 présente une courbure moins importante que la surface postérieure 15 de manière à former un maximum d'épaisseur Emax entre la patte proximale 12 et la plaque distale 11, au niveau de la zone de courbure 19.

Au moins une partie de la patte proximale 12 est d'épaisseur constante à partir du bord proximal 17. De même, au moins une partie de la plaque distale 11 est préférentiellement d'épaisseur constante à partir du bord distal 16. Il est également possible d'envisager que l'épaisseur du corps principal 13 soit plus élevée au voisinage du bord distal 16 et/ou du bord proximal 17 que dans les zones situées entre le maximum d'épaisseur Emax et lesdits voisinages (non représenté).

En tout état de cause, la zone de courbure 19 et en particulier le maximum d'épaisseur Emax sont destinés à être placés au niveau du trait de fracture 8 tel qu'illustré aux figures 10 et 11, afin de présenter une rigidité maximale au niveau dudit trait de fracture 8 pour assurer un maintien en position fiable des corps osseux 2, 3.

De façon préférentielle, une fenêtre traversante 28 est ménagée au travers du corps principal 13 au niveau de la zone de courbure 19, afin de permettre en particulier l'insertion d'un matériau ostéo-inducteur dans le corps du patient, et en particulier au niveau du trait de fracture 8, au travers de ladite zone de courbure 19 de l'implant chirurgical 1, par exemple dans le but de réparer une fracture ou une fêlure des corps osseux 2, 3. La fenêtre traversante 28 est avantageusement destinée à être placée à l'aplomb du trait de fracture 8.

De façon préférentielle, les bords latéraux 18 sont sensiblement symétriques l'un par rapport à l'autre vis-à-vis d'un plan médian du corps principal 13. Selon cette configuration préférentielle, le corps principal 13 est lui-même de forme générale sensiblement symétrique. Bien entendu, sans sortir du cadre de l'invention, on pourra envisager au contraire que le corps principal 13 soit asymétrique, et forme par exemple une deuxième courbure autour de la direction sagittale.

Tel qu'illustré aux figures, les bords latéraux 18 sont de préférence incurvés et divergent l'un de l'autre depuis l'extrémité libre de la patte proximale 12 jusqu'à l'extrémité libre opposée de la plaque distale 11, de sorte que la patte proximale 12 est prolongée par la plaque distale 11 en s'évasant de façon progressive. L'implant chirurgical présente ainsi avantageusement la forme générale d'un « *Y* ».

Dans la variante de l'invention représentée aux figures, et en particulier aux figures 3 à 5 et 10, ledit bord distal 16 est avantageusement biseauté du côté de la surface postérieure 15 de manière à présenter un congé postérieur 20. La présence de ce congé postérieur 20 a la double fonction :
- de conférer une certaine souplesse au bord distal 16 propre à réduire la douleur subie par le patient sur le corps osseux 3 correspondant, préférentiellement l'extrémité distale du radius, et
- de conférer au bord distal 16 une forme qui suit la courbure externe extrémale du corps osseux 3, de manière à créer une continuité de forme de nature à limiter le risque d'abrasion ou de coupure du corps du patient par le bord distal 16, par exemple des tissus du poignet du patient, des tendons, ou des nerfs.

De préférence, une portion au moins des bords latéraux 18 est biseautée du côté de la surface antérieure 14 à partir du bord distal 16, de manière à former des congés antérieurs 21. La présence des congés antérieurs 21 permet à la plaque distale 11 d'épouser sensiblement la forme du corps osseux 3, avantageusement la portion extrémale du radius, afin de réduire la douleur subie par le patient et de s'adapter à des morphologies osseuses variées.

Dans le but de s'adapter finement à la morphologie osseuse du patient, et notamment de la partie extrémale de l'os et des corps osseux 2, 3, le bord distal 16 présente avantageusement une portion ulnaire 22 et une portion radiale 23, cette dernière étant en retrait par rapport à ladite portion ulnaire 22 de manière à pouvoir épouser la ligne anatomique des corps osseux 2, 3, en particulier un os du radius du patient. De préférence, le bord distal 16 s'étend transversalement et est composé de deux portions successives, la portion ulnaire 22 avancée par rapport à la portion radiale 23. Le bord distal 16 présente ainsi avantageusement une forme en « S » pour limiter la douleur subie par le patient. On entend par « *portion ulnaire* » la partie du bord distal 16 destinée à être disposée préférentiellement du côté ulnaire de l'avant-bras, c'est-à-dire sensiblement dans le prolongement de l'auriculaire. La portion radiale est préférentiellement destinée à être disposée du côté radial de l'avant bras, opposé au côté ulnaire, le côté radial étant sensiblement dans le prolongement du pouce.

Ainsi, l'implant chirurgical 1 se présente sous la forme de deux variantes principales formées par un implant pour avant-bras gauche (non représenté aux figures) et un implant pour avant bras droit (représenté aux figures).

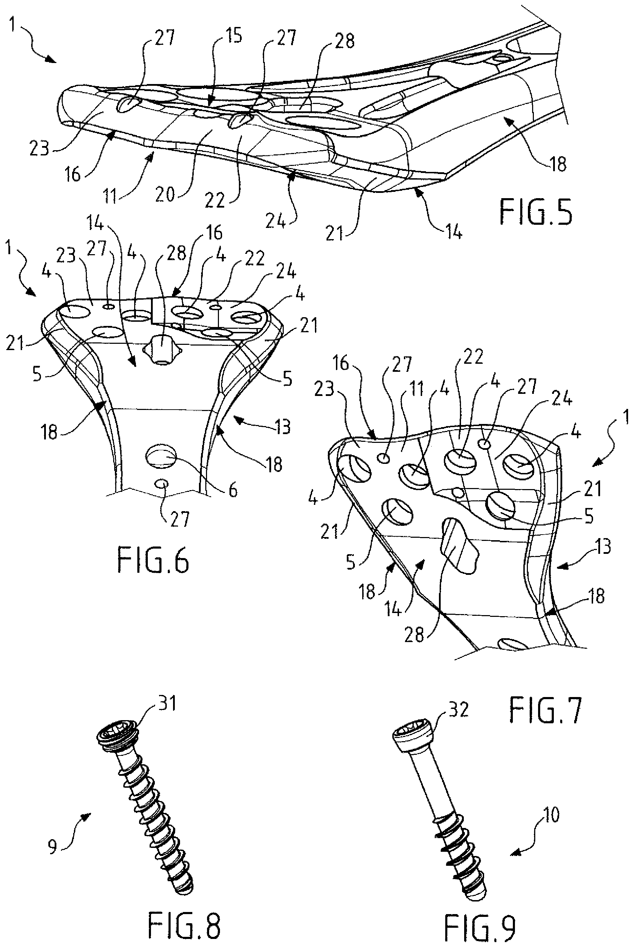

Tel qu'illustré en particulier aux figures 6 et 7, la plaque distale 11 comporte de préférence une concavité 24 ménagée dans la surface antérieure 14 et s'étendant à partir de la portion ulnaire 22 dudit bord distal 16. La présence de cette forme en creux qu'est la concavité 24 permet au corps principal d'épouser le mieux possible la forme physiologique osseuse afin de limiter la douleur subie par le patient et de s'adapter à la morphologie de ce dernier.

De préférence, le corps principal 13 est pourvu d'au moins un sillon longitudinal, de préférence deux sillons longitudinaux 25, 26, ménagé au niveau de la zone de courbure 19 du corps principal 13, tels que représentés par exemple aux figures 1 à 3 et 11. Les sillons longitudinaux 25, 26 sont préférentiellement disposés en « V » de façon parallèle aux bords latéraux 18. Leur orientation longitudinale leur permet, tout en permettant de préserver sensiblement les propriétés mécaniques en flexion du corps principal liées à l'épaisseur de la zone de courbure 19 décrites précédemment, d'alléger le corps principal 13 afin de faciliter sa fabrication par moulage. Les sillons longitudinaux 25, 26 sont avantageusement et ménagés dans la surface postérieure 15 du corps principal 13 qui n'est pas destinée à être en contact avec les corps osseux 2, 3.

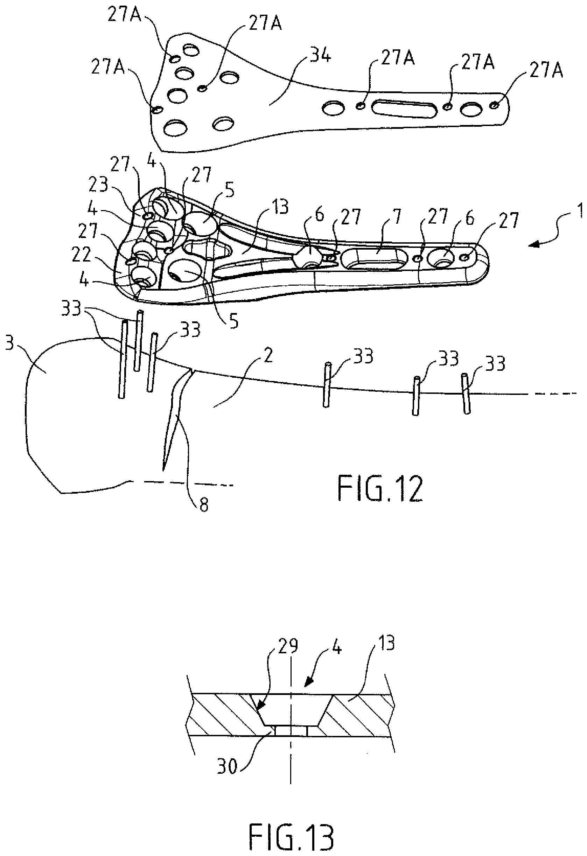

Tel qu'illustré notamment à la figure 12, de façon préférentielle, l'implant chirurgical 1 comprend des trous de guidage 27 traversants ménagés dans le corps principal 13, l'implant chirurgical 1 étant destiné à être enfilé par l'intermédiaire desdits trous de guidage 27 sur des broches de guidage 33 lors de sa mise en place dans le corps du patient. Les broches de guidage 33 sont par exemple implantées dans les corps osseux 2, 3 préalablement à la mise en place de l'implant chirurgical 1 en lui-même, et destinées à être retirées après la mise en place dudit implant chirurgical 1. La mise en place et le positionnement de l'implant chirurgical 1 est ainsi particulièrement aisée et précise.

Les figures illustrent par ailleurs un exemple de réalisation préféré des moyens de fixation 4, 5, 6, 7.

De façon préférentielle, le corps principal 13 est pourvu d'au moins une ouverture de vissage distale 4, 5 et au moins une ouverture de vissage proximale 6, qui sont ménagées respectivement dans la plaque distale 11 et dans la patte proximale 12, et qui forment ou contribuent à former les moyens de fixation 4, 5, 6, 7.

Les ouvertures de vissage sont préférentiellement destinées à accueillir chacune une vis de fixation 9, 10 de l'implant chirurgical 1 sur les corps osseux 2, 3, et sont orientées de façon à induire un positionnement divergent desdites vis de fixation 9, 10 dans le corps principal 13, c'est-à-dire en berceau, afin d'assurer la fiabilité de la fixation et du maintien de l'implant chirurgical 1.

De préférence, au moins trois ouvertures de vissage distales 4, de préférence quatre, sont ménagées dans le corps principal 13 et disposées à la même distance les unes que les autres du bord distal 16, les ouvertures de vissage distales 4 formant, ou contribuant à former, les moyens de fixation 4, 5, 6, 7.

Selon cette configuration préférentielle, les ouvertures de vissage distales 4 présentent un alignement sensiblement transversal et de nature à suivre la courbe physiologique du corps osseux 3, en particulier formé par l'extrémité du radius, dans laquelle les vis de fixation 9, 10 correspondantes sont destinées à être implantées. Un tel alignement permet au corps principal 13 de se déformer légèrement en limitant la douleur subie par le patient, tout en assurant un maintien optimal de l'implant sur le corps osseux 3.

Dans la variante préférentielle illustrée aux figures, le corps principal 13 est pourvu de quatre ouvertures de vissage distales 4 alignées le long du bord distal 16, de deux ouvertures de vissage distales 5 intermédiaires réparties dans la plaque distale 11 de façon transversale et à l'écart du bord distal 16, à proximité de la fenêtre traversante 28. Dans cette variante préférentielle, le corps principal 13 est également pourvu de deux ouvertures de vissage proximales 6, chacune d'elle étant placée à une extrémité de la patte proximale 12 de manière à être longitudinalement alignées.

Tel que représenté aux figures, la patte proximale 12 est pourvue d'une ouverture de vissage oblongue 7 longitudinale étant destinée à recevoir une vis de fixation 9, 10 de l'implant chirurgical 1 sur le corps osseux 2, 3 tout en autorisant le coulissage de ladite vis de fixation 9, 10 le long de ladite ouverture de vissage oblongue 7, en particulier lorsque ladite vis de fixation 9, 10 n'est pas ou peu serrée. Grâce à cette conception préférée, le chirurgien peut opérer un réglage de la proximité des corps osseux 2, 3 lorsque seule l'ouverture de vissage oblongue 7 est occupée par une vis de fixation 9 pour la patte proximale 12 (les ouvertures de vissage proximales 6 étant alors dépourvues de vis), en faisant coulisser ladite vis de fixation 9, 10 dans l'ouverture de vissage oblongue 7.

Tel qu'illustré à la figure 13, au moins l'une des ouvertures de vissage 4, 5, 6, 7, de préférence toutes les ouvertures de vissages 4, 5, 6, 7, forme avantageusement un cône de rétention 29 la vis par l'intermédiaire de la tête de cette dernière, le cône de rétention 29 se prolongeant par un col intérieur 30 formant une surface d'appui sous-tête de la vis de fixation 9, 10. Le cône de rétention 29 axial de l'ouverture de vissage 4, 5, 6, 7, permet, lors du serrage de la vis de fixation 9, 10, d'exercer une pression radiale sur la tête 31, 32 de cette dernière, afin de prévenir sensiblement tout risque de dévissage de ladite vis de fixation 9, 10, et tout mouvement de l'implant chirurgical 1 par rapport aux corps osseux 2, 3. Le col intérieur 30 permet quant à lui de donner une orientation à la vis de fixation 9, 10, en l'espèce de manière à ce que les vis de fixation 9, 10 soient orientées en berceau, de façon divergentes lorsqu'elles sont en place au sein du corps principal 13.

Par exemple, pour fixer on pourra utiliser à la fois :
- au moins une vis de fixation 9 à tête filetée 31 (telle qu'illustrée par exemple à la figure 8), les filets de sa tête filetée 31 étant destinés à être vissés dans le cône de rétention 29, et
- au moins une vis de fixation 10 de serrage, à tête conique non filetée 32 (telle qu'illustrée par exemple à la figure 9), permettant de serrer solidement l'implant chirurgical 1 contre les corps 2, 3 osseux du patient.

Les vis de fixation 9, 10 représentées à titre d'exemple aux figures présentent un diamètre nominal de 2, 7 mm, une tête cylindro-conique, une extrémité de pénétration arrondie, et une empreinte de tête en étoile de type « *Torx* ». Bien entendu, d'autres types de vis de fixation pourront être utilisés sans sortir du cadre de l'invention.

L'invention concerne également en tant que tel un kit chirurgical incluant un implant chirurgical 1 tel que décrit ci-avant, et également un sabot 34 (tel qu'illustré par exemple à la figure 12) de forme complémentaire à la plaque distale 11, de manière à pouvoir être rapporté de façon amovible sur ladite plaque distale 11, par exemple au cours de la mise en place de l'implant chirurgical 1 dans le corps d'un patient.

Le kit chirurgical comprend également de façon avantageuse les broches de guidage 33 décrites ci-avant, ainsi que les vis de fixation 9, 10.

Le sabot 34 est prévu pour être déposé temporairement, lors de la mise en place de l'implant chirurgical 1 dans le corps du patient, sur la surface postérieure 15 de ce dernier.

Le sabot 34 est avantageusement pourvu de trous de guidage 27A correspondant aux trous de guidage 27 de l'implant chirurgical 1, de manière à ce que les trous de guidage 27A puissent être enfilés sur les broches de guidage 33 en se superposant aux trous de guidage 27 de l'implant chirurgical.

De la même façon, le sabot 34 présente avantageusement des ouvertures de perçage 4A, 5A, 6A, 7A conçues pour se superposer et correspondantes aux ouvertures de vissage 4, 5, 6, 7 de l'implant chirurgical 1 lorsque le sabot 34 est rapporté sur ce dernier.

De cette façon, lorsque l'implant chirurgical 1 est positionné à l'aide des broches de guidage 33 sur les corps osseux 2, 3 du patient, il est possible d'effectuer un perçage ou pré-perçage des corps osseux 2, 3 en vue de l'insertion des vis de fixation 9, 10. Le pré-perçage ou perçage étant effectué à travers les ouvertures de perçage 4A, 5A, 6A, 7A, l'intégrité des ouvertures de vissage 4, 5, 6, 7 est préservée, et l'opération de mise en place de l'implant chirurgical facilitée.

Par ailleurs, le sabot 34 est préférentiellement réalisé en un matériau radio-visible, de préférence métallique, par exemple de l'inox ou du titane. Lors de la mise en place de l'implant chirurgical 1, il est ainsi possible de connaître précisément le positionnement de ce dernier dans le corps du patient en dépit de sa radio-transparence préférentielle (notamment lorsqu'il est réalisé en PEEK). En effet, le sabot 34 étant visible à la radiographie et rapporté directement sur ledit implant chirurgical 1, le chirurgien peut parfaitement estimer le positionnement de l'implant chirurgical 1 à l'aide d'une radiographie.

Il est décrit ci-après en tant que tel un procédé de mise en place d'un implant chirurgical 1 tel que décrit ci-avant, à l'aide d'un kit chirurgical décrit ci-avant.

Le procédé de mise en place de l'implant chirurgical comprend avantageusement les étapes successives suivantes :
- on incise le corps du patient afin de pouvoir introduire l'implant chirurgical 1 à l'intérieur, au niveau des corps osseux 2, 3 à traiter,
- on implante des broches de guidage 33 dans les corps osseux 2, 3, par exemple par impaction, et optionnellement avec l'aide de l'implant chirurgical 1 ou du sabot 34, les broches de guidage 33 pouvant être impactées dans les corps osseux 2, 3 au travers des trous de guidage 27 et 27A de ces derniers,
- on positionne l'implant chirurgical 1 sur les corps osseux 2, 3 à l'aide des broches de guidage 33, de façon à ce que la surface antérieure 14 repose au moins partiellement sur les corps osseux 2, 3, la plaque distale 11 étant en contact avec une partie extrémale de l'os à traiter formant le corps osseux 3, et la patte proximale 12 étant en contact avec une partie intermédiaire ou médiane de l'os à traiter formant le corps osseux 2,
- on rapporte le sabot 34 sur l'implant chirurgical 1,
- on effectue une radiographie pour vérifier le bon positionnement de l'implant chirurgical 1 radio-transparent sur les corps osseux, en visualisant le sabot 34 radio-visible,
- on effectue un pré-perçage des corps osseux au travers de tout ou partie des ouvertures de perçage 4A, 5A, 6A, 7A du sabot 34,
- on retire le sabot 34,
- on insère une première vis de fixation 10 de serrage dans l'une des ouvertures de vissage distales 4 de la plaque distale 11, et une deuxième vis de fixation 10 de serrage dans l'ouverture de vissage oblongue 7 de la patte proximale 12,
- avant de serrer lesdites première et deuxième vis de fixation de serrage 10, on règle la proximité et l'orientation des corps osseux 2, 3 en faisant coulisser la deuxième vis de fixation 10 le long de l'ouverture de vissage oblongue 7,
- on serre la première et la deuxième vis de fixation de serrage 10,
- on insère ensuite les vis de fixation 9 à tête filetée 31 dans tout ou partie des ouvertures de vissage 4, 5, 6 restantes, afin d'assurer une fixation durable de l'implant chirurgical 1 dans le corps du patient,
- au fur et à mesure des opérations d'insertion et de serrage des vis de fixation 9, 10, on retire les broches de guidage 33 des corps osseux,
- on referme l'incision du corps du patient.

Bien entendu, certaines étapes pourront être inversées sans sortir du cadre de l'invention.

En définitive, l'implant chirurgical 1 ainsi mis en place de façon aisée, précise et rapide, permet une ostéosynthèse particulièrement rapide des corps osseux 2,3, et de bonne qualité, tout en limitant la douleur subie par le patient, quelle que soit sa morphologie.

### POSSIBILITE D'APPLICATION INDUSTRIELLE

L'invention trouve son application industrielle dans la conception, la réalisation et la mise en oeuvre d'implants chirurgicaux d'ostéosynthèse, en particulier conçus pour le traitement de fractures ou de fêlures de corps osseux, notamment de l'avant-bras, du tibia ou du pied.

## Revendications

1. - Implant chirurgical (1) destiné à être rapporté sur des corps osseux (2, 3) d'un patient par l'intermédiaire de moyens de fixation (4, 5, 6, 7) afin de maintenir en position lesdits corps osseux (2, 3) les uns par rapport aux autres, ledit implant chirurgical (1) comportant un corps principal (13) formé par une plaque distale (11) qui se prolonge par une patte proximale (12), la plaque distale (11) s'étendant sensiblement selon un plan d'extension distal (Pd), la patte proximale (12) s'étendant sensiblement le long d'un plan d'extension proximal (Pp) sécant au plan d'extension distal (Pd), la patte proximale (12) et la plaque distale (11) étant reliées par une zone de courbure (19), ladite zone de courbure (19) présentant une épaisseur la plus élevée du corps principal (13), l'implant chirurgical (1) étant **caractérisé en ce que** la zone de courbure (19) présente une épaisseur supérieure aux épaisseurs respectives de la plaque distale (11) et de la patte proximale (12).

2. - Implant chirurgical (1) selon la revendication précédente, **caractérisé en ce que** la plaque distale (11) est inclinée par rapport à la patte proximale (12), de sorte que le plan d'extension distal (Pd) et le plan d'extension proximal (Pp) sont sécants l'un par rapport à l'autre de manière à former un angle d'élévation (α) étant compris entre 10° et 80°, de préférence entre 20° et 30°, et de façon encore plus préférentielle d'environ 25°.

3. - Implant chirurgical (1) selon l'une quelconque des revendications précédentes, **caractérisé en ce que** le corps principal (13) est pourvu d'au moins un sillon longitudinal, de préférence deux sillons longitudinaux (25, 26), ménagé au niveau de la zone de courbure (19) du corps principal (13).

4. - Implant chirurgical (1) selon l'une quelconque des revendications précédentes, **caractérisé en ce que** ledit corps principal (13) s'étend longitudinalement entre un bord distal (16) à partir duquel s'étend la plaque distale (11), et un bord proximal (17) à partir duquel s'étend la patte proximale (12), et transversalement entre deux bords latéraux (18), et **en ce que** la zone de courbure (19) comporte un maximum d'épaisseur (Emax), l'épaisseur de la zone de courbure (19) étant décroissante à partir du maximum d'épaisseur (Emax), d'une part en direction de la plaque distale (11), d'autre part en direction de la patte proximale (12), le maximum d'épaisseur (Emax) étant destiné à être placé au niveau d'un trait de fracture (8) des corps osseux (2, 3).

5. - Implant chirurgical (1) selon la revendication précédente, **caractérisé en ce que** le corps principal (13) présente :
- une surface antérieure (14) de forme générale convexe destinée à reposer au moins partiellement sur lesdits corps osseux (2, 3), et
- une surface postérieure (15) de forme générale concave opposée à la surface antérieure (14), et présentant une courbure moins importante que la surface postérieure (15) de manière à former ledit maximum d'épaisseur (Emax) entre la patte proximale (12) et la plaque distale (11), au niveau de la zone de courbure (19).

6. - Implant chirurgical (1) selon la revendication précédente, **caractérisé en ce que** le bord distal (16) présente une portion ulnaire (22) et une portion radiale (23), cette dernière étant en retrait par rapport à ladite portion ulnaire (22) de manière à pouvoir épouser la ligne anatomique des corps osseux (2, 3).

7. - Implant chirurgical (1) selon la revendication précédente, **caractérisé en ce que** la plaque distale (11) comporte une concavité (24) ménagée dans la surface antérieure (14) et s'étendant à partir de la portion ulnaire (22) dudit bord distal (16).

8. - Implant chirurgical (1) selon l'une quelconque des revendications 4 à 7, **caractérisé en ce qu'**au moins trois ouvertures de vissage distales (4), de préférence quatre, sont ménagées dans le corps principal (13) et disposées à la même distance les unes que les autres du bord distal (16), les ouvertures de vissage distales (4) formant, ou contribuant à former, les moyens de fixation (4, 5, 6, 7).

9. - Implant chirurgical (1) selon l'une quelconque des revendications précédentes, **caractérisé en ce qu'**une fenêtre traversante (28) est ménagée au travers du corps principal (13) au niveau de la zone de courbure (19), afin de permettre en particulier l'insertion d'un matériau ostéo-inducteur dans le corps du patient au travers de ladite zone de courbure (19) de l'implant chirurgical (1), par exemple dans le but de réparer une fracture ou une fêlure des corps osseux (2, 3).

10. - Implant chirurgical (1) selon l'une quelconque des revendications précédentes, **caractérisé en ce que** le corps principal (13) est pourvu d'au moins une ouverture de vissage distale et au moins une ouverture de vissage proximale, qui sont ménagées respectivement dans la plaque distale (11) et dans la patte proximale (12), et qui forment ou contribuent à former les moyens de fixation (4, 5, 6, 7), lesdites ouvertures de vissage étant destinées à accueillir chacune une vis de fixation (9, 10) de l'implant chirurgical (1) sur les corps osseux (2, 3), les ouvertures de vissage étant orientées de façon à induire un positionnement divergent desdites vis de fixation (9, 10) dans le corps principal (13).

11. - Implant chirurgical (1) selon la revendication précédente, **caractérisé en ce qu'**au moins l'une des ouvertures de vissage (4, 5, 6, 7) forme un cône de rétention (29) de la vis par l'intermédiaire de la tête de cette dernière, le cône de rétention (29) se prolongeant par un col intérieur formant une surface d'appui sous-tête de la vis de fixation (9, 10).

12. - Implant chirurgical (1) selon l'une quelconque des revendications précédentes, **caractérisé en ce qu'**il comprend des trous de guidage (27) traversants ménagés dans le corps principal (13), l'implant chirurgical (1) étant destiné à être enfilé par l'intermédiaire desdits trous de guidage (27) sur des broches de guidage (33) lors de sa mise en place dans le corps du patient.

13. - Implant chirurgical (1) selon l'une quelconque des revendications précédentes, **caractérisé en ce que** le corps principal (13) forme une seule pièce monobloc réalisée en un matériau radio-transparent, de préférence du polyéther-éther-cétone (PEEK), ce dernier étant optionnellement chargé en carbone.

14. - Kit chirurgical incluant un implant chirurgical (1) selon l'une quelconque des revendications précédentes, et étant **caractérisé en ce qu'**il inclut également un sabot (34) de forme complémentaire à la plaque distale (11), de manière à pouvoir être rapporté de façon amovible sur ladite plaque distale (11), par exemple au cours de la mise en place de l'implant chirurgical (1) dans le corps d'un patient.

## Patentansprüche

1. - Chirurgisches Implantat (1), das dazu bestimmt ist, durch Befestigungsmittel (4, 5, 6, 7) an Knochenkörpern (2, 3) eines Patienten angebracht zu werden, um die besagten Knochenkörper (2, 3) relativ zueinander in Position zu halten, wobei das chirurgische Implantat (1) einen Hauptkörper (13) umfasst, der von einer distalen Platte (11) gebildet wird, die sich in einer proximalen Lasche (12) fortsetzt, wobei sich die distale Platte (11) im Wesentlichen entlang einer distalen Verlängerungsebene (Pd) erstreckt, wobei sich die proximale Lasche (12) im Wesentlichen entlang einer proximalen Verlängerungsebene (Pp) erstreckt, die die distale Verlängerungsebene (Pd) schneidet, wobei die proximale Lasche (12) und die distale Platte (11) durch eine Krümmungszone (19) verbunden sind, wobei die Krümmungszone (19) eine größte Dicke des Hauptkörpers (13) aufweist, wobei das chirurgische Implantat (1) **dadurch gekennzeichnet ist, dass** die Krümmungszone (19) eine Dicke aufweist, die größer als die jeweiligen Dicken der distalen Platte (11) und der proximalen Lasche (12) ist.

2. - Chirurgisches Implantat (1) nach dem vorhergehenden Anspruch, **dadurch gekennzeichnet, dass** die distale Platte (11) in Bezug auf die proximale Lasche (12) geneigt ist, so dass die distale Verlängerungsebene (Pd) und die proximale Verlängerungsebene (Pp) einander schneiden, um einen Elevationswinkel (α) zu bilden, der zwischen 10° und 80°, vorzugsweise zwischen 20° und 30°, und noch bevorzugter etwa 25° beträgt.

3. - Chirurgisches Implantat (1) nach einem der vorhergehenden Ansprüche, **dadurch gekennzeichnet, dass** der Hauptkörper (13) mit mindestens einer Längsrille, vorzugsweise zwei Längsrillen (25, 26), versehen ist, die im Bereich der Krümmungszone (19) des Hauptkörpers (13) ausgebildet sind.

4. - Chirurgisches Implantat (1) nach einem der vorhergehenden Ansprüche, **dadurch gekennzeichnet, dass** sich der Hauptkörper (13) in Längsrichtung zwischen einem distalen Rand (16), von dem aus sich die distale Platte (11) erstreckt, und einem proximalen Rand (17), von dem aus sich die proximale Lasche (12) erstreckt, und quer zwischen zwei seitlichen Rändern (18) erstreckt, und dass die Krümmungszone (19) ein Dickenmaximum (Emax) aufweist, wobei die Dicke der Krümmungszone (19) ausgehend vom Dickenmaximum (Emax) einerseits in Richtung der distalen Platte (11) und andererseits in Richtung der proximalen Lasche (12) abnimmt, wobei das Dickenmaximum (Emax) dazu bestimmt ist, auf der Höhe einer Bruchlinie (8) der Knochenkörper (2, 3) angeordnet zu werden.

5. - Chirurgisches Implantat (1) nach einem der vorhergehenden Ansprüche, **dadurch gekennzeichnet, dass** der Hauptkörper (13) aufweist:
- eine vordere Oberfläche (14) von allgemein konvexer Form, die dazu bestimmt ist, zumindest teilweise an den Knochenkörpern (2, 3) zu ruhen, und
- eine hintere Oberfläche (15) von allgemein konkaver Form, die der vorderen Oberfläche (14) gegenüberliegt und eine geringere Krümmung als die hintere Oberfläche (15) aufweist, um das Dickenmaximum (Emax) zwischen der proximalen Lasche (12) und der distalen Platte (11) auf Höhe der Krümmungszone (19) zu bilden.

6. - Chirurgisches Implantat (1) nach dem vorhergehenden Anspruch, **dadurch gekennzeichnet, dass** der distale Rand (16) einen ulnaren Abschnitt (22) und einen radialen Abschnitt (23) aufweist, wobei letzterer in Bezug auf den ulnaren Abschnitt (22) zurückversetzt ist, um sich an die anatomische Linie der Knochenkörper (2, 3) anpassen zu können.

7. - Chirurgisches Implantat (1) nach dem vorhergehenden Anspruch, **dadurch gekennzeichnet, dass** die distale Platte (11) eine Konkavität (24) aufweist, die in der vorderen Oberfläche (14) ausgebildet ist und sich von dem ulnaren Abschnitt (22) des distalen Randes (16) aus erstreckt.

8. - Chirurgisches Implantat (1) nach einem der Ansprüche 4 bis 7, **dadurch gekennzeichnet, dass** mindestens drei, vorzugsweise vier, distale Schraubenöffnungen (4) im Hauptkörper (13) ausgebildet und im gleichen Abstand zueinander von dem distalen Rand (16) angeordnet sind, wobei die distalen Schraubenöffnungen (4) die Befestigungsmittel (4, 5, 6, 7) bilden oder dazu beitragen, diese zu bilden.

9. - Chirurgisches Implantat (1) nach einem der vorhergehenden Ansprüche, **dadurch gekennzeichnet, dass** ein Durchgangsfenster (28) durch den Hauptkörper (13) an der Krümmungszone (19) vorgesehen ist, um insbesondere das Einführen eines osteoinduktiven Materials in den Körper des Patienten durch die Krümmungszone (19) des chirurgischen Implantats (1) zu ermöglichen, beispielsweise zum Zweck der Reparatur eines Bruchs oder eines Risses der Knochenkörper (2, 3).

10. - Chirurgisches Implantat (1) nach einem der vorhergehenden Ansprüche, **dadurch gekennzeichnet, dass** der Hauptkörper (13) mit mindestens einer distalen Schraubenöffnung und mindestens einer proximalen Schraubenöffnung versehen ist, welche in der distalen Platte (11) bzw. in der proximalen Lasche (12) ausgebildet sind und welche die Befestigungsmittel (4, 5, 6, 7) bilden oder dazu beitragen, diese zu bilden, wobei die Schraubenöffnungen dazu bestimmt sind, jeweils eine Schraube (9, 10) zur Befestigung des chirurgischen Implantats (1) an den Knochenkörpern (2, 3) aufzunehmen, wobei die Schraubenöffnungen so ausgerichtet sind, dass sie eine divergente Positionierung der Befestigungsschrauben (9, 10) in dem Hauptkörper (13) induzieren.

11. - Chirurgisches Implantat (1) nach dem vorhergehenden Anspruch, **dadurch gekennzeichnet, dass** mindestens eine der Schraubenöffnungen (4, 5, 6, 7) einen Retentionskonus (29) für die Schraube über deren Kopf bildet, wobei der Retentionskonus (29) in einen inneren Hals übergeht, der eine Unterkopfauflagefläche für die Befestigungsschraube (9, 10) bildet.

12. - Chirurgisches Implantat (1) nach einem der vorhergehenden Ansprüche, **dadurch gekennzeichnet, dass** es durchgehende Führungslöcher (27) im Hauptkörper (13) aufweist, wobei das chirurgische Implantat (1) dazu bestimmt ist, beim Einsetzen in den Körper des Patienten über die Führungslöcher (27) auf Führungsstifte (33) aufgefädelt zu werden.

13. - Chirurgisches Implantat (1) nach einem der vorhergehenden Ansprüche, **dadurch gekennzeichnet, dass** der Hauptkörper (13) ein einziges einteiliges Teil bildet, das aus einem strahlendurchlässigen Material, vorzugsweise Polyetheretherketon (PEEK), hergestellt ist, wobei letzteres optional mit Kohlenstoff gefüllt ist.

14. - Chirurgisches Set, das ein chirurgisches Implantat (1) nach einem der vorhergehenden Ansprüche einschließt und **dadurch gekennzeichnet ist, dass** es auch einen Schuh (34) einschließt, dessen Form komplementär zu der distalen Platte (11) ist, so dass er abnehmbar an der distalen Platte (11) angebracht werden kann, beispielsweise während des Einsetzens des chirurgischen Implantats (1) in den Körper eines Patienten.

## Claims

1. - A surgical implant (1) intended to be attached on bone bodies (2, 3) of a patient via fastening means (4, 5, 6, 7) in order to hold in position said bone bodies (2, 3) relative to each other, said surgical implant (1) including a main body (13) formed by a distal plate (11) which is extended by a proximal tab (12), the distal plate (11) extending substantially according to a distal extension plane (Pd), the proximal tab (12) extending substantially along a proximal extension plane (Pp) intersecting the distal extension plane (Pd), the proximal tab (12) and the distal plate (11) being linked by a bending area (19), the bending area (19) having a highest thickness in the main body (13), the surgical implant (1) being **characterized in that** the bending area (19) has a thickness greater than the respective thicknesses of the distal plate (11) and of the proximal tab (12).

2. - The surgical implant (1) according to the preceding claim, **characterized in that** the distal plate (11) is inclined relative to the proximal tab (12), so that the distal extension plane (Pd) and the proximal extension plane (Pp) are intersecting each other so as to form an angle of elevation (α) being comprised between 10° and 80°, preferably between 20° and 30°, and even more preferably about 25°.

3. - The surgical implant (1) according to any one of the preceding claims, **characterized in that** the main body (13) is provided with at least one longitudinal furrow, preferably two longitudinal furrows (25, 26), arranged at the bending area (19) of the main body (13).

4. - The surgical implant (1) according to any one of the preceding claims, **characterized in that** the main body (13) extends longitudinally between a distal edge (16) from which extends the distal plate (11), and a proximal edge (17) from which extends the proximal tab (12), and transversely between two side edges (18), and **in that** the bending area (19) includes a maximum thickness (Emax), the thickness of the bending area (19) being decreasing from the maximum thickness (Emax), on the one hand, in the direction of the distal plate (11), and on the other hand, in the direction of the proximal tab (12), the maximum thickness (Emax) being intended to be placed at a fracture line (8) of bone bodies (2, 3).

5. - The surgical implant (1) according to the preceding claim, **characterized in that** the main body (13) has:
- an anterior surface (14) with a convex general shape intended to rest at least partially on said bone bodies (2, 3), and
- a posterior surface (15) with a concave general shape opposite to the anterior surface (14), and having a bend less significant than the posterior surface (15) so as to form the maximum thickness (Emax) between the proximal tab (12) and the distal plate (11), at the bending area (19).

6. - The surgical implant (1) according to the preceding claim, **characterized in that** the distal edge (16) has an ulnar portion (22) and a radial portion (23), the latter being recessed relative to said ulnar portion (22) so as to conform to the anatomical line of the bone bodies (2, 3).

7. - The surgical implant (1) according to the preceding claim, **characterized in that** the distal plate (11) has a concavity (24) arranged in the anterior surface (14) and extending from the ulnar portion (22) of said distal edge (16).

8. - The surgical implant (1) according to any one of claims 4 to 7, **characterized in that** at least three distal screw openings (4), preferably four, are arranged in the main body (13) and disposed at the same distance as each other from the distal edge (16), the distal screw openings (4) forming, or contributing to form, the fastening means (4, 5, 6, 7).

9. - The surgical implant (1) according to any one of the preceding claims, **characterized in that** a through window (28) is arranged through the main body (13) at the bending area (19), in order to allow in particular the insertion of an osteoinductive material in the patient's body through said bending area (19) of the surgical implant (1), for example with the aim of repairing a fracture or a crack of the bone bodies (2, 3).

10. - The surgical implant (1) according to any one of the preceding claims, **characterized in that** the main body (13) is provided with at least one distal screw-in opening and at least one proximal screw opening (6), which are arranged respectively in the distal plate (11) and in the proximal tab (12), and which form or contribute to form the fastening means (4, 5, 6, 7), said screw openings being intended to receive, each, a fastening screw (9, 10) of the surgical implant (1) on the bone bodies (2, 3), the screw openings being oriented so as to induce a divergent positioning of said fastening screws (9, 10) in the main body (13).

11. - The surgical implant (1) according to the preceding claim, **characterized in that** at least one of the screw openings (4, 5, 6, 7) forms a retention cone (29) of the screw via the head of the latter, the retention cone (29) extending by an inner collar forming a sub-head bearing surface of the fastening screw (9, 10).

12. - The surgical implant (1) according to any one of the preceding claims, **characterized in that** it comprises through guide holes (27) arranged in the main body (13), the surgical implant (1) being intended to be threaded via said guide holes (27) on guide pins (33) during its setting up in the patient's body.

13. - The surgical implant (1) according to any one of the preceding claims, **characterized in that** the main body (13) forms a one piece and unitary part made of a radiolucent material, preferably polyether ether ketone (PEEK), the latter being optionally loaded with carbon.

14. - A surgical kit including a surgical implant (1) according to any one of the preceding claims, and being **characterized in that** it also includes a shoe (34) complementary in shape to the distal plate (11), so as to be removably attachable on said distal plate (11), for example during the setting up of the surgical implant (1) in a patient's body.
